# EUROPEAN PATENT APPLICATION

(11) **EP 4 393 586 A1**
(43) Date of publication of application: **03.07.2024**
(21) Application number: 21967479.3
(22) Date of filing: 13.12.2021
(51) Int. Cl.: B01J 23/89, B01J 35/00, B01J 35/10, C07C 1/22, C07C 15/085

(54) **DIMETHYL BENZYL ALCOHOL HYDROGENOLYSIS CATALYST, AND PREPARATION METHOD THEREFOR AND USE THEREOF**

(71) Applicant: Wanhua Chemical Group Co., Ltd, Yantai Shandong 264000 (CN)
(72) Inventor: LI, Zuojin, Yantai, Shandong 264000 (CN); ZHAN, Jishan, Yantai, Shandong 264000 (CN); YU, Haibo, Yantai, Shandong 264000 (CN); SHA, Yu, Yantai, Shandong 264000 (CN); SUN, Kang, Yantai, Shandong 264000 (CN); WANG, Tongji, Yantai, Shandong 264000 (CN); YE, Fei, Yantai, Shandong 264000 (CN); WANG, Leilei, Yantai, Shandong 264000 (CN); MENG, Meng, Yantai, Shandong 264000 (CN); WANG, Qinlong, Yantai, Shandong 264000 (CN); LI, Yuan, Yantai, Shandong 264000 (CN)
(74) Representative: Cabinet Laurent & Charras
(86) International application number: PCT/CN2021/137424
(87) International publication number: WO 2023/108336

(57) **Abstract**

The present application provides a dimethyl benzyl alcohol hydrogenolysis catalyst, and a preparation method therefor and a use thereof. Active components of the dimethyl benzyl alcohol hydrogenolysis catalyst comprise Pd, Ru, Cu, and Ni, Pd and Ru in the active components are highly dispersed on a surface layer of the catalyst, thereby facilitating inhibiting the occurrence of side reactions such as excessive hydrogenation, and dimethyl benzyl alcohol diffused into a catalyst pore channel can be subjected to hydrogenolysis reaction under the catalysis of Cu and Ni to generate cumene while further hydrogenation is difficult to occur to generate a by-product isopropyl cyclohexane; a support of the dimethyl benzyl alcohol hydrogenolysis catalyst is a composite support comprising ZnO, SiO₂, and Al₂O₃, thereby facilitating obtaining an efficient hydrogenolysis catalyst which has active components highly dispersed and is reliable in strength and moderate in acidity; the dimethyl benzyl alcohol hydrogenolysis catalyst of the present application has the advantages of being high in active component dispersity, smooth in catalyst pore channel and the like, and has excellent activity and selectivity when being used for preparing cumene by means of dimethyl benzyl alcohol hydrogenolysis.

## Description

### TECHNICAL FIELD

The present application relates to the technical field of hydrogenolysis catalysts, and specifically relates to a dimethylphenylcarbinol hydrogenolysis catalyst and a preparation method therefor and use thereof.

### BACKGROUND

The industrial methods for the production of propylene oxide (PO) mainly include chlorohydrination, hydrogen-peroxide direct oxidation, and co-oxidation (the Halcon method). At present, chlorohydrination is the main route for PO production, but this process has serious problems such as equipment corrosion and environmental pollution. The route of hydrogen-peroxide direct oxidation requires highly costly raw materials, and its economy is adversely affected. The co-oxidation method is to react organic peroxides with propylene to produce propylene oxide. For the conventional ethylbenzene co-oxidation method and isobutene co-oxidation method, although the serious environmental pollution brought by chlorohydrination which have a high investment and long process is avoided, a large number of by-products are co-produced during the PO production process, and the production cost of PO is greatly affected by the price fluctuation of co-products.

Isopropylbenzene co-oxidation (PO-CHP process) includes three core reactions of isopropylbenzene peroxidation, propylene epoxidation, and dimethylphenylcarbinol hydrogenolysis as well as related separation processes, wherein isopropylbenzene hydrogen peroxide is used as the oxygen source, the co-produced dimethylphenylcarbinol is converted to isopropylbenzene by hydrogenolysis, and the isopropylbenzene is returned to the peroxidation unit to react to generate isopropylbenzene hydrogen peroxide, realizing the cyclic utilization of isopropylbenzene. Compared with other processes, the isopropylbenzene co-oxidation method has the advantages of extremely high conversion rate and selectivity, short process route, low equipment investment, no co-products, and more stable economic benefits.

Dimethylphenylcarbinol hydrogenolysis is one of the core reactions of PO-CHP process. Dimethylphenylcarbinol hydrogenolysis catalysts mainly include platinum-palladium precious metal catalysts, nickel-based catalysts, and copper-based catalysts, etc., which have been disclosed in many patents.

American patent US3337646 proposes a method for preparing isopropylbenzene by gas-phase hydrogenolysis of α,α-dimethylphenylcarbinol, which employs a Ni-Cr-AL₂O₃ catalyst; due to the containing of Cr, the catalyst has a serious problem of environmental pollution in the preparation, use, and disposal processes.

Patent CN1308273C discloses a method for preparing isopropylbenzene by catalytic hydrogenolysis of α,α-dimethylphenylcarbinol; this patent employs 2wt% Pd-C catalyst, the catalyst is costly, and the reaction needs to introduce substances such as halogenated aromatic hydrocarbons, sodium formate, formic acid, and indole, which increases the difficulty of separation and cost.

Patent CN104230640A discloses a method for preparing isopropylbenzene by α,α-dimethylphenylcarbinol hydrogenolysis; this patent employs a Pd-Ni/SiO₂ catalyst modified by Mg/Ca/Ba; the selectivity of isopropylbenzene generation by hydrogenolysis is generally lower than 98.5%, and the cost of catalyst is high and the selectivity is low.

Patent CN104874406 discloses a Pt-loaded hydrogenolysis catalyst, which employs phenolic resin-based activated carbon as a carrier; the catalyst preparation process is complicated, and also difficult to scale up for production; the catalyst has significantly reduced selectivity after 300 hours of operation, showing poor stability.

Patent CN1257138C proposes a method of H₂-CO gas mixture reduction Cu catalyst; the catalyst used is still a Cu-Cr catalyst, and the catalyst stability index is not disclosed in this patent.

Patent CN101992098 discloses a Cu-Zn-Al catalyst for dimethylphenylcarbinol hydrogenolysis to prepare isopropylbenzene; the space velocity employed in this patent is 1.5 h⁻¹, the space velocity is low, and the condition of the catalyst after use and the strength of the catalyst are not disclosed in this patent.

Patent CN112569930A discloses a preparation method for isopropylbenzene and isopropylbenzene obtained therefrom; in this patent, Pd and a carbon-containing precursor are loaded onto an aluminum oxide carrier, the residual carbon content after roasting is less than 1.5%, the Pd loading content is 0.5%, and the cost of the catalyst is high. In addition, the moulding method of the catalyst and condition of the catalyst particles after reaction are not mentioned in this patent.

The conventional precious metal catalysts have the disadvantages of high catalyst cost, easily causing saturation of the aromatic ring, and poor selectivity of isopropylbenzene when used in dimethylphenylcarbinol hydrogenolysis; the nickel-based and copper-based catalysts have the disadvantages of low activity, poor selectivity, easy to be sintered, and poor liquid resistance.

At present, the catalysts prepared by the existing technology have the problems of high loading content of precious metals such as Pt/Pd, low activity, poor selectivity, poor high-temperature stability, poor liquid-resistance, and serious environmental pollution when used in dimethylphenylcarbinol catalytic hydrogenolysis for the preparation of isopropylbenzene. Therefore, it is of great significance to develop a hydrogenolysis catalyst with excellent hydrogenolysis performance.

### SUMMARY

The present application provides a dimethylphenylcarbinol hydrogenolysis catalyst and a preparation method therefor and use thereof. Active components of the dimethylphenylcarbinol hydrogenolysis catalyst comprise Pd, Ru, Cu, and Ni; Pd and Ru of the active components are highly dispersed on the surface layer of the catalyst, which is beneficial to inhibit the occurrence of side reactions such as excessive hydrogenation, and dimethylphenylcarbinol diffused into the inside of pores of the catalyst can be subjected to hydrogenolysis under the catalytic effect of Cu and Ni to generate isopropylbenzene, and, however, can hardly be further hydrogenated to generate the by-product isopropylcyclohexane; a carrier of the dimethylphenylcarbinol hydrogenolysis catalyst is a composite carrier including ZnO, SiO₂, and Al₂O₃, which is conducive to the preparation of an efficient hydrogenolysis catalyst with highly dispersed active components, reliable strength, and moderate acidity; the dimethylphenylcarbinol hydrogenolysis catalyst in the present application has the advantages of highly dispersed active components and unobstructed pores, and has excellent activity and selectivity when used in isopropylbenzene preparation by the dimethylphenylcarbinol hydrogenolysis, and also has good liquid resistance and high stability.

In a first aspect, the present application provides a dimethylphenylcarbinol hydrogenolysis catalyst, wherein provided that the total weight of the dimethylphenylcarbinol hydrogenolysis catalyst is 100wt% and calculated based on inorganic oxides, the dimethylphenylcarbinol hydrogenolysis catalyst comprises the following components:

| | |
|---|---|
| PdO | 0.01-0.3wt%; |
| RuO₂ | 0.05-0.8wt%; |
| CuO | 15.0-25.0wt%; |
| ZnO | 15.0-25.0wt%; |
| NiO | 2.0-10.0wt%; |
| Al₂O₃ | 15.0-40.0wt%; and |
| SiO₂ | 20.0-45.0wt%. |

Active components of the dimethylphenylcarbinol hydrogenolysis catalyst in the present application comprise Pd, Ru, Cu, and Ni; Pd and Ru of the active components are highly dispersed on the surface layer of the catalyst, which is beneficial to inhibit the occurrence of side reactions such as excessive hydrogenation, and dimethylphenylcarbinol diffused into the inside of pores of the catalyst can be subjected to hydrogenolysis under the catalytic effect of Cu and Ni to generate isopropylbenzene, and, however, can hardly be further hydrogenated to generate the by-product isopropylcyclohexane; a carrier of the dimethylphenylcarbinol hydrogenolysis catalyst is a composite carrier including ZnO, SiO₂, and Al₂O₃, which is conducive to the preparation of an efficient hydrogenolysis catalyst with highly dispersed active components, reliable strength, and moderate acidity.

Provided that the total weight of the dimethylphenylcarbinol hydrogenolysis catalyst is 100wt% and calculated based on inorganic oxides, the dimethylphenylcarbinol hydrogenolysis catalyst comprises 0.01-0.3wt% PdO, such as 0.01wt%, 0.05wt%, 0.1wt%, 0.15wt%, 0.2wt%, 0.25wt%, or 0.3wt%; however, the proportion is not limited to the listed values, and other unlisted values within the numerical range are also appropriate.

Provided that the total weight of the dimethylphenylcarbinol hydrogenolysis catalyst is 100wt% and calculated based on inorganic oxides, the dimethylphenylcarbinol hydrogenolysis catalyst comprises 0.05-0.8wt% RuO₂, such as 0.05wt%, 0.1wt%, 0.2wt%, 0.3wt%, 0.4wt%, 0.5wt%, 0.6wt%, 0.7wt%, or 0.8wt%; however, the proportion is not limited to the listed values, and other unlisted values within the numerical range are also appropriate.

Provided that the total weight of the dimethylphenylcarbinol hydrogenolysis catalyst is 100wt% and calculated based on inorganic oxides, the dimethylphenylcarbinol hydrogenolysis catalyst comprises 15.0-25.0wt% CuO, such as 15.0wt%, 17.0wt%, 19.0wt%, 20.0wt%, 21.0wt%, 23.0wt%, or 25.0wt%; however, the proportion is not limited to the listed values, and other unlisted values within the numerical range are also appropriate.

Provided that the total weight of the dimethylphenylcarbinol hydrogenolysis catalyst is 100wt% and calculated based on inorganic oxides, the dimethylphenylcarbinol hydrogenolysis catalyst comprises 15.0-25.0wt% ZnO, such as 15.0wt%, 17.0wt%, 19.0wt%, 20.0wt%, 21.0wt%, 23.0wt%, or 25.0wt%; however, the proportion is not limited to the listed values, and other unlisted values within the numerical range are also appropriate.

Provided that the total weight of the dimethylphenylcarbinol hydrogenolysis catalyst is 100wt% and calculated based on inorganic oxides, the dimethylphenylcarbinol hydrogenolysis catalyst comprises 2.0-10.0wt% NiO, such as 2.0wt%, 4.0wt%, 5.0wt%, 6.0wt%, 7.0wt%, 8.0wt%, or 10.0wt%; however, the proportion is not limited to the listed values, and other unlisted values within the numerical range are also appropriate.

Provided that the total weight of the dimethylphenylcarbinol hydrogenolysis catalyst is 100wt% and calculated based on inorganic oxides, the dimethylphenylcarbinol hydrogenolysis catalyst comprises 15.0-40.0wt% Al₂O₃, such as 15.0wt%, 20.0wt%, 25.0wt%, 30.0wt%, 35.0wt%, or 40.0wt%; however, the proportion is not limited to the listed values, and other unlisted values within the numerical range are also appropriate.

Provided that the total weight of the dimethylphenylcarbinol hydrogenolysis catalyst is 100wt% and calculated based on inorganic oxides, the dimethylphenylcarbinol hydrogenolysis catalyst comprises 20.0-45.0wt% SiO₂, such as 20.0wt%, 25.0wt%, 30.0wt%, 35.0%, 40.0wt%, or 45.0wt%; however, the proportion is not limited to the listed values, and other unlisted values within the numerical range are also appropriate.

As a preferred technical solution of the present application, in the dimethylphenylcarbinol hydrogenolysis catalyst, a molar ratio of Pd to Ru is 1: (1-10), a molar ratio of Cu to Zn is 1: (0.5-1.0), and a molar ratio of Cu to Ni is 1: (0.1-0.5).

In the dimethylphenylcarbinol hydrogenolysis catalyst in the present application, the molar ratio of Pd to Ru is 1: (1-10). When the content of Pd is higher and the content of Ru is lower, the cost of the catalyst is higher and excessive hydrogenation is serious; when the content of Pd is lower and the content of Ru is higher, the activity of the catalyst is insufficient. The molar ratio of Cu to Zn is 1: (0.5-1.0), and the molar ratio of Cu to Ni is 1: (0.1- 0.5). When the content of Cu is higher, the dispersion of Cu is poor, and in turn affects the catalyst activity; when the content of Cu is lower, the catalyst activity is insufficient; the introduction of suitable content of Zn in the catalyst can significantly improve the dispersion of Cu; when the content of Ni is higher, the catalyst activity is high but the selectivity is poor; when the content of Ni is lower, the catalyst activity is low. Therefore, the suitable contents of Pd, Ru, Cu, Ni and Zn are beneficial to prepare a hydrogenolysis catalyst with high activity and selectivity.

As a preferred technical solution of the present application, the dimethylphenylcarbinol hydrogenolysis catalyst has a hollow ring shape with an outer diameter of 3-5 mm, such as 3 mm, 3.5 mm, 4 mm, 4.5 mm, or 5 mm; an inner diameter of 1-3 mm, such as 1 mm, 1.5 mm, 2 mm, 2.5 mm, or 3 mm; and a length of 3-8 mm, such as 3 mm, 4 mm, 5 mm, 6 mm, 7 mm, or 8 mm; however, the sizes are not limited to the listed values, and other unlisted values within the numerical ranges are also appropriate.

The dimethylphenylcarbinol hydrogenolysis catalyst in the present application has a hollow ring shape, which improves the mass transfer performance of the catalyst, reduces the occurrence of side reactions, and at the same time contributes to a low bed pressure drop.

In a second aspect, the present application provides a preparation method for the dimethylphenylcarbinol hydrogenolysis catalyst described in the first aspect, the preparation method comprising the following steps:
(1) preparing a mixed solution 1 of a copper-containg compound, a zinc-containing compound, and a nickel-containing compound; adding colloidal silica to an aqueous urea solution to obtain a mixed solution 2; adding an aminosilane coupling agent to an alcoholic nanosilica solution to obtain a mixed solution 3; and preparing a mixed solution 4 of a palladium-containing compound and a ruthenium-containing compound;
(2) adding the mixed solution 1 to the mixed solution 2, sequentially performing stirring, evaporating to dry, and roasting, and mixing a composite metal compound obtained with an aluminum oxide powder and an extrusion promoter to obtain a mixed powder;
(3) adding the mixed solution 4 dropwise to the mixed solution 3 to obtain a mixed solution 5; and
(4) adding the mixed powder of step (2) to the mixed solution 5 of step (3), and sequentially performin mixing, extrusion moulding, drying, and roasting to obtain the dimethylphenylcarbinol hydrogenolysis catalyst;
wherein steps (2) and (3) are not required to be performed in a particular order.

For the preparation method in the present application, the composite metal compound containing copper, zinc, and nickel is first prepared, then mixed with the aluminum oxide powder and extrusion promoter to obtain a mixed powder, whereby the active components Cu and Ni can be uniformly dispersed inside the composite carrier comprising ZnO, SiO₂, and Al₂O₃; therefore, the dimethylphenylcarbinol diffused into the inside of the pores of the catalyst can be subjected to hydrogenolysis under the catalytic effect of Cu and Ni to generate isopropylbenzene, and, however, can hardly be further hydrogenated to generate the by-product isopropylcyclohexane; subsequently, the impregnation solution (mixed solution 5) containing Pd and Ru and the aforementioned mixed powder are sequentially mixed, extrusion-moulded, dried, and roasted, so that the Pd and Ru of the active components are highly dispersed in the surface layer of the catalyst, which is beneficial to inhibit the occurrence of side reactions such as excessive hydrogenation.

In addition, the preparation process of the mixed solution 5 is especially defined for the preparation method in the present application, i.e., the mixed solution 4 containing the palladium compound and the ruthenium compound is added dropwise to an alcoholic nanosilica solution containing the aminosilane coupling agent (mixed solution 3); the use of alcoholic nanosilica solution is conducive to a high dispersion degree of palladium and ruthenium.

Moreover, for the preparation method in the present application, two sources are designed for the SiO₂ in the composite carrier, i.e., the colloidal silica in the mixed solution 2 and the alcoholic nanosilica solution in the mixed solution 3. The SiO₂ introduced in the form of alkaline colloidal silica accounts for 20-40wt% of the total amount of SiO₂ in the catalyst; the colloidal silica in the mixed solution 2 is able to promote the dispersion of Cu, Zn, and Ni, and the alcoholic nanosilica solution in the mixed solution 3 is able to improve the dispersion degree of Pd and Ru; the two sources are complementary to each other and conducive to improving the hydrogenolysis catalyst activity.

As a preferred technical solution of the present application, in the mixed solution 1 of step (1), the copper-containing compound comprises copper formate and/or copper acetate.

Preferably, in the mixed solution 1 of step (1), the zinc-containing compound comprises zinc formate and/or zinc acetate.

Preferably, in the mixed solution 1 of step (1), the nickel-containing compound comprises nickel formate and/or nickel acetate.

Preferably, in the mixed solution 1 of step (1), metal ions have a molar concentration of 1.0-2.0 mol/L, such as 1.0 mol/L, 1.1 mol/L, 1.3 mol/L, 1.5 mol/L, 1.7 mol/L, 1.9 mol/L, or 2.0 mol/L; however, the molar concentration is not limited to the listed values, and other unlisted values within the numerical range are also appropriate.

In the present application, the metal formate and/or metal acetate is used as the raw material of mixed solution 1, and accordingly, the subsequent slurry obtained from the reaction between the metal salt in the mixed solution 1 and urea in the mixed solution 2 does not need to be washed and can be directly dried by evaporation and roasted; moreover, the formate group and/or acetate group will be thermally decomposed during the roasting process, which improves the porosity of the dimethylphenylcarbinol hydrogenolysis catalyst and improves the mass transfer performance of the dimethylphenylcarbinol hydrogenolysis catalyst.

Preferably, the aqueous urea solution of step (1) has a mass concentration of 15-30wt%, such as 15wt%, 18wt%, 20wt%, 23wt%, 25wt%, 26wt%, 28wt%, or 30wt%; however, the mass concentration is not limited to the listed values, and other unlisted values within the numerical range are also appropriate.

Preferably, the colloidal silica of step (1) is an alkaline silica sol.

Preferably, for the alkaline silica sol, a content of SiO₂ is 30-40wt%, such as 30wt%, 31wt%, 33wt%, 35wt%, 38wt%, or 40wt%; a particle size is 20-40 nm, such as 20 nm, 25 nm, 30 nm, 35 nm, or 40 nm; and a pH is 8.0-10.0, such as 8.0, 8.5, 9.0, 9.5, or 10.0; however, the parameters are not limited to the listed values, and other unlisted values within the numerical ranges are also appropriate.

Preferably, for the alcoholic nanosilica solution of step (1), a content of SiO₂ is 15-20wt%, such as 15wt%, 16wt%, 17wt%, 18wt%, 19wt%, or 20wt%; and a particle size is 15-30 nm, such as 15 nm, 18 nm, 20 nm, 23 nm, 25 nm, 28 nm, or 30 nm; however, the parameters are not limited to the listed values, and other unlisted values within the numerical ranges are also appropriate.

The preparation method in the present application employs the alcoholic nanosilica solution, which is conducive to a high dispersion degree of Pd and Ru; furthermore, the content of SiO₂ in the alcoholic nanosilica solution is limited to 15-20wt%, and thus the content of SiO₂ in the alcoholic nanosilica solution is avoided from being overly high, which is unfavourable to the dispersion of Pd and Ru, and also from being overly low, which is unfavourable to the moulding of catalyst and may lead to low strength of the moulded catalyst.

Preferably, an alcoholic solvent in the alcoholic nanosilica solution of step (1) comprises any one or a combination of at least two of methanol, ethanol, or propanol, and typical but non-limiting examples of the combination comprise a combination of methanol and ethanol, a combination of methanol and propanol, or a combination of ethanol and propanol.

As a preferred technical solution of the present application, the aminosilane coupling agent of step (1) comprises any one or a combination of at least two of γ-aminopropyltrimethoxysilane, γ-aminopropyltriethoxysilane, anilinomethyltrimethoxysilane, or anilinomethyltriethoxysilane, and typical but non-limiting examples of the combination comprise: a combination of γ-aminopropyltrimethoxysilane and γ-aminopropyltriethoxysilane, a combination of γ-aminopropyltriethoxysilane and anilinomethyltrimethoxysilane, or a combination of anilinomethyltrimethoxysilane and anilinomethyltriethoxysilane.

Preferably, in the preparation for the mixed solution 3 of step (1), a mass ratio of the aminosilane coupling agent to the alcoholic nanosilica solution is controlled to be 1: (20-50), such as 1: 20, 1: 25, 1: 30, 1: 35, 1: 40, 1: 45, or 1: 50; however, the mass ratio is not limited to the listed values, and other unlisted values within the numerical range are also appropriate.

The addition of aminosilane coupling agent in the preparation method in the present application not only improves the dispersion degree of Pd and Ru and enhances the hydrogenolysis reaction activity of dimethylphenylcarbinol, but also reduces the formation of unfavourable substances such as water to a certain extent during the reaction, reduces the menace to the active components and carrier, and is advantageous for the stability of catalyst; and the mass ratio of the aminosilane coupling agent to the alcoholic nanosilica solution is further limited to 1: (20-50), which not only avoids the amount of aminosilane coupling agent being too low to improve the dispersion of Pd and Ru, but also avoids the amount of aminosilane coupling agent being too high and adversely affecting the diffusion of raw material and product.

Preferably, in the mixed solution 4 of step (1), the palladium-containing compound comprises palladium acetylacetonate.

Preferably, in the mixed solution 4 of step (1), the ruthenium-containing compound comprises ruthenium acetylacetonate.

The preparation method in the present application employs palladium acetylacetonate and ruthenium acetylacetonate as the palladium source and the ruthenium source, respectively, which, compared with palladium chloride/palladium nitrate and ruthenium chloride/ruthenium nitrate, are more conducive to improving the dispersion degree of Pd and Ru, thereby improves the reaction activity of the dimethylphenylcarbinol hydrogenolysis catalyst.

Preferably, a solvent in the mixed solution 4 of step (1) comprises any one or a combination of at least two of benzene, toluene, or chloroform, and typical but non-limiting examples of the combination comprise: a combination of benzene and toluene, a combination of toluene and chloroform, or a combination of benzene and chloroform.

As a preferred technical solution of the present application, in step (2), a molar ratio of the metal ions in the mixed solution 1 to urea in the mixed solution 2 is controlled to be 1: (2.0-3.0), such as 1: 2.0, 1: 2.2, 1: 2.4, 1: 2.5, 1: 2.7, 1: 2.9, or 1: 3.0; however, the molar ratio is not limited to the listed values, and other unlisted values within the numerical range are also appropriate.

For the preparation method in the present application, the amount of urea is limited, so as to avoid the amount of urea being too low to improve the porosity of the catalyst, and also to avoid the amount of urea being too high which results in that the catalyst has a small bulk density and has a low content of active sites per unit volume.

Preferably, the evaporating to dry of step (2) is performed at a temperature of 80-100°C, such as 80°C, 85°C, 90°C, 95°C, or 100°C; however, the temperature is not limited to the listed values, and other unlisted values within the numerical range are also appropriate..

Preferably, the evaporating to dry of step (2) is performed for a period of 4-24 h, such as 4 h, 8 h, 12 h, 16 h, 20 h, or 24 h; however, the period is not limited to the listed values, and other unlisted values within the numerical range are also appropriate.

Preferably, the roasting of step (2) is performed at a temperature of 250-400°C, such as 250°C, 280°C, 300°C, 320°C, 350°C, 380°C, or 400°C; however, the temperature is not limited to the listed values, and other unlisted values within the numerical range are also appropriate.

Preferably, the roasting of step (2) is performed for a period of 2-8 h, such as 2 h, 3 h, 4 h, 5 h, 6 h, 7 h, or 8 h; however, the period is not limited to the listed values, and other unlisted values within the numerical range are also appropriate.

As a preferred technical solution of the present application, the aluminum oxide powder of step (2) has a particle size of 80-150 mesh, such as 80 mesh, 90 mesh, 100 mesh, 110 mesh, 120 mesh, 130 mesh, 140 mesh, or 150 mesh; however, the particle size is not limited to the listed values, and other unlisted values within the numerical range are also appropriate.

For the preparation method in the present application, the addition of the aluminum oxide powder with suitable particle size can avoid the overly coarse aluminum oxide powder being detrimental to the dispersion of aluminum oxide and also avoid the overly fine aluminum oxide powder being detrimental to the diffusion properties of the moulded catalyst.

Preferably, an amount of the aluminum oxide powder of step (2) is 15-40wt% of a total mass of the composite metal compound and the aluminum oxide powder, such as 15wt%, 20wt%, 25wt%, 30wt%, 35wt%, or 40wt%; however, the amount is not limited to the listed values, and other unlisted values within the numerical range are also appropriate.

For the preparation method in the present application, the addition of a suitable content of aluminum oxide powder can provide the catalyst with a suitable acidity, which can avoid insufficient catalyst activity brought by fewer acidic sites, and at the same time avoid dehydration polymerization of dimethylphenylcarbinol brought by excessive acidic sites, which will further affect the selectivity of the catalyst.

Preferably, the extrusion promoter of step (2) comprises sesbania powder.

Preferably, an amount of the extrusion promoter of step (2) is 2-5wt% of a total mass of the composite metal compound and the aluminum oxide powder, such as 2wt%, 2.5wt%, 3wt%, 3.5wt%, 4wt%, 4.5wt%, or 5wt%; however, the amount is not limited to the listed values, and other unlisted values within the numerical range are also appropriate.

As a preferred technical solution of the present application, process conditions for the extrusion moulding of step (4) comprise: mixing and kneading materials used for moulding thoroughly, and performing extrusion moulding at room temperature using an F-26 twin-screw extruder.

Preferably, the extrusion moulding has an extrusion pressure of 100-200 N, such as 100 N, 120 N, 150 N, 180 N, or 200 N, and a screw rotating speed of 10-50 r/min, such as 10 r/min, 20 r/min, 30 r/min, 40 r/min, or 50 r/min; however, the parameters are not limited to the listed values, and other unlisted values within the numerical ranges are also appropriate.

Preferably, the drying of step (4) is performed at a temperature of 100-120°C, such as 100°C, 105°C, 110°C, 115°C, or 120°C; however, the temperature is not limited to the listed values, and other unlisted values within the numerical range are also appropriate.

Preferably, the drying of step (4) is performed for a period of 4-12 h, such as 4 h, 8 h, 12 h, 16 h, 20 h, or 24 h; however, the period is not limited to the listed values, and other unlisted values within the numerical range are also appropriate.

Preferably, the roasting of step (4) is performed at a temperature of 300-450°C, such as 300°C, 330°C, 350°C, 380°C, 400°C, 430°C, or 450°C; however, the temperature is not limited to the listed values, and other unlisted values within the numerical range are also appropriate.

Preferably, the roasting of step (4) is performed for a period of 2-8 h, such as 2 h, 3 h, 4 h, 5 h, 6 h, 7 h, or 8 h; however, the period is not limited to the listed values, and other unlisted values within the numerical range are also appropriate.

As a preferred technical solution of the present application, the preparation method comprises the following steps:
(1) preparing solutions:
   preparing a mixed solution 1 of a copper-containing compound, a zinc-containing compound, and a nickel-containing compound; the copper-containing compound comprises copper formate and/or copper acetate, the zinc-containing compound comprises zinc formate and/or zinc acetate, and the nickel-containing compound comprises nickel formate and/or nickel acetate; and metal ions in the mixed solution 1 have a molar concentration of 1.0-2.0 mol/L;
   adding alkaline colloidal silica to an aqueous urea solution having a mass concentration of 15-30wt% to obtain a mixed solution 2; for the alkaline silica sol, a content of SiO₂ is 30-40wt%, a particle size is 20-40 nm, and a pH is 8.0-10.0;
   adding an aminosilane coupling agent to an alcoholic nanosilica solution, wherein a mass ratio of the aminosilane coupling agent to the alcoholic nanosilica solution is controlled to be 1: (20-50), to obtain a mixed solution 3; for the alcoholic nanosilica solution, a content of SiO₂ is 15-20wt% and a particle size is 15-30 nm; an alcoholic solvent in the alcoholic nanosilica solution comprises any one or a combination of at least two of methanol, ethanol, or propanol; the aminosilane coupling agent comprises any one or a combination of at least two of γ-aminopropyltrimethoxysilane, γ-aminopropyltriethoxysilane, anilinomethyltrimethoxysilane, or anilinomethyltriethoxysilane;
   preparing a mixed solution 4 of a palladium-containing compound and a ruthenium-containing compound; the palladium-containing compound comprises palladium acetylacetonate, the ruthenium-containing compound comprises ruthenium acetylacetonate, and a solvent in the mixed solution 4 comprises any one or a combination of at least two of benzene, toluene, or chloroform;
(2) adding the mixed solution 1 to the mixed solution 2, and stirring the mixture, wherein a molar ratio of the metal ions in the mixed solution 1 to urea in the mixed solution 2 is controlled to be 1:(2.0-3.0), firstly evaporating the mixture at 80-100°C for 4-24 h and then roasting at 250-400°C for 2-8 h, and mixing a composite metal compound obtained with an aluminum oxide powder having a particle size of 80-150 mesh and an extrusion promoter to obtain a mixed powder; an amount of the aluminum oxide powder is 15-40wt% of a total mass of the composite metal compound and the aluminum oxide powder, and an amount of the extrusion promoter is 2-5wt% of a total mass of the composite metal compound and the aluminum oxide powder;
(3) adding the mixed solution 4 dropwise to the mixed solution 3 to obtain a mixed solution 5; and
(4) adding the mixed powder of step (2) to the mixed solution 5 of step (3) and stirring them, mixing and kneading materials used for moulding thoroughly, and performing extrusion moulding at room temperature using an F-26 twin-screw extruder, wherein the extrusion moulding is performed under an extrusion pressure controlled at 100-200 N and a screw rotating speed controlled at 10-50 r/min, and firstly drying at 100-120°C for 4-12 h and then roasting at 300-450°C for 2-8 h to obtain the dimethylphenylcarbinol hydrogenolysis catalyst;
wherein steps (2) and (3) are not required to be performed in a particular order.

In a third aspect, the present application provides use of a dimethylphenylcarbinol hydrogenolysis catalyst, wherein the dimethylphenylcarbinol hydrogenolysis catalyst described in the first aspect or the dimethylphenylcarbinol hydrogenolysis catalyst obtained by the preparation method described in the second aspect is applied to a reaction of preparing isopropylbenzene by dimethylphenylcarbinol hydrogenolysis; refer to CN104230642B for the specific process.

Compared with the prior art, the present application has at least the following beneficial effects:
the dimethylphenylcarbinol hydrogenolysis catalyst in the present application has the advantages of highly dispersed active components and unobstructed pores, and has excellent activity and selectivity when used in isopropylbenzene preparation by the dimethylphenylcarbinol hydrogenolysis, and also has good liquid resistance and high stability.

### DETAILED DESCRIPTION

To facilitate understanding the present application, examples of the present application are described below. It should be apparent to those skilled in the art that the examples are merely used for a better understanding of the present application and should not be regarded as a specific limitation to the present application.

### <Source of raw materials>

Isopropylbenzene, purchased from Shanghai Aladdin Biochemical Technology Co., Ltd.;
Dimethylphenylcarbinol, purchased from TCI Development Co., Ltd.;
Sodium-type colloidal silica, purchased from Linyi Kehan Silicon Products Co., Ltd.;
Palladium acetylacetonate, purchased from Shanghai Aladdin Biochemical Technology Co., Ltd.;
Ruthenium acetylacetonate, purchased from Shanghai Aladdin Biochemical Technology Co., Ltd.;
Copper formate, purchased from Shanghai Aladdin Biochemical Technology Co., Ltd.;
Zinc formate, purchased from Shanghai Aladdin Biochemical Technology Co., Ltd.;
Nickel formate, purchased from Shanghai Aladdin Biochemical Technology Co., Ltd.;
Urea, purchased from Shanghai Aladdin Biochemical Technology Co., Ltd.;
Alcoholic silica solution, Xuancheng Jingrui New Materials Co., Ltd.; and
Aluminum oxide powder, purchased from Shanghai Aladdin Biochemical Technology Co., Ltd.

### <Test method>

1. Composition analysis of dimethylphenylcarbinol hydrogenolysis catalyst is analyzed by an X-ray fluorescence spectrometry (XRF);
2. Dimethylphenylcarbinol conversion rate = (1 - molar amount of dimethylphenylcarbinol remaining in the reaction solution / molar amount of dimethylphenylcarbinol contained in the raw material)* 100%;

Isopropylbenzene selectivity = molar amount of isopropylbenzene generated / molar amount of dimethylphenylcarbinol that has been converted * 100%;
wherein the molar amount of dimethylphenylcarbinol contained in the raw material, the molar amount of isopropylbenzene generated, and the molar amount of dimethylphenylcarbinol remaining in the reaction solution are analyzed and calculated by an 7820A gas chromatography from Agilent; the test conditions are as follows: the DB-5 column and FID detector are used, the temperature of vaporization chamber is 260°C, the temperature of detector is 260 °C, and the carrier gas is high-purity N₂ at a flow rate of 30 mL/min.

### Example 1

This example provides a preparation method for a dimethylphenylcarbinol hydrogenolysis catalyst, and the preparation method comprises the following steps:
(1) Solution preparation:
   To a reaction kettle, 672.4 g of water was first added, and then 96.4 g of copper formate, 24.7 g of nickel formate, and 85.6 g of zinc formate were added, and stirred thoroughly to dissolve to obtain a mixed solution 1;
   133.3 g of urea was dissolved in 755.2 g of water to obtain an aqueous urea solution, and then 78.0 g of alkaline colloidal silica (with a SiO₂ content of 30wt%, a particle size of 30 nm, and a pH of 9.0) was added and stirred thoroughly to obtain a mixed solution 2;
   11.6 g of γ-aminopropyltrimethoxysilane was added to 253.5 g of an ethanol nanosilica solution (with a SiO₂ content of 20wt%, and a particle size of 30 nm) and stirred thoroughly to obtain a mixed solution 3;
   0.5 g of palladium acetylacetonate and 4.2 g of ruthenium acetylacetonate were dissolved in 93.8 g of benzene and stirred thoroughly to obtain a mixed solution 4;
(2) the mixed solution 1 was added to the mixed solution 2 and stirred, the mixture was first dried by evaporation at 95°C for 12 h, and then roasted at 300°C for 6 h, and the obtained composite metal compound was mixed with 40.0 g of an aluminum oxide powder having a particle size of 100 mesh and 4.3 g of sesbania powder to obtain a mixed powder; in this step, an amount of the aluminum oxide powder was 27.8wt% of a total mass of the composite metal compound and the aluminum oxide powder, and an amount of the extrusion promoter was 3.0wt% of a total mass of the composite metal compound and the aluminum oxide powder;
(3) the mixed solution 4 was added dropwise to the mixed solution 3 to obtain a mixed solution 5; and
(4) the mixed powder of step (2) was added to the mixed solution 5 of step (3) and stirred, and the materials required for moulding were mixed and kneaded sufficiently and subjected to extrusion moulding at room temperature by an F-26 twin-screw extruder, wherein the extrusion moulding was performed under an extrusion pressure controlled at 150 N and a screw rotating speed controlled at 30 r/min, and firstly, the extruded materials were dried at 110°C for 4 h, and then roasted at 450°C for 4 h, so as to obtain the dimethylphenylcarbinol hydrogenolysis catalyst denoted as Catalyst A;
in the preparation method, steps (2) and (3) were not required to be performed in a particular order.

According to the X-ray fluorescence spectrometry (XRF) analysis, the composition of Catalyst A is (calculated based on inorganic oxides): 0.10wt% PdO, 0.70wt% RuO₂, 17.0wt% CuO, 5.0wt% NiO, 18.2wt% ZnO, 20.0wt% Al₂O₃, and 39.0wt% SiO₂.

### Catalyst reduction:

The Catalyst A was loaded in a fixed-bed hydrogenation reactor with a catalyst loading volume of 100 mL. The catalyst was reduced in a gas mixture of nitrogen and hydrogen before use, and the volumetric space velocity of the gas mixture was maintained at 300 h⁻¹ during the reduction process; firstly, the temperature of the reactor was raised to 160°C and held for 2 h to remove physical water adsorbed by the catalyst, then the gas mixture of hydrogen and nitrogen containing Hz by a volume fraction of 5v% was introduced for pre-reduction for 1 h, and then the proportion of hydrogen in the gas mixture of hydrogen and nitrogen was gradually increased to 10v%, 20v%, 50v%, and 100v%; during the process, the hot-spot temperature of the catalyst bed was controlled to be no more than 250°C, and finally the catalyst bed was raised to 250°C and reduced in pure hydrogen atmosphere for 4 h.

### Catalyst performance evaluation:

The raw material was an isopropylbenzene solution containing 25wt% of dimethylphenylcarbinol, and reacted at a pressure of 2.0 MPa, a temperature of 150°C, a Hz/alcohol molar ratio of 8: 1, and a liquid hourly space velocity of 3 h⁻¹. The result of the hydrogenolysis reaction is shown in Table 1.

### Example 2

This example provides a preparation method for a dimethylphenylcarbinol hydrogenolysis catalyst, and the preparation method comprises the following steps:
(1) Solution preparation:
   To a reaction kettle, 672.4 g of water was first added, and then 124.8 g of copper formate, 12.4 g of nickel formate, and 75.0 g of zinc formate were added, and stirred thoroughly to dissolve to obtain a mixed solution 1;
   145.9 g of urea was dissolved in 583.5 g of water to obtain an aqueous urea solution, and then 83.6 g of alkaline colloidal silica (with a SiO₂ content of 40wt%, a particle size of 40 nm, and a pH of 9.0) was added and stirred thoroughly to obtain a mixed solution 2;
   6.5 g of γ-aminopropyltriethoxysilane was added to 264.0 g of an ethanol nanosilica solution (with a SiO₂ content of 20wt%, and a particle size of 30 nm) and stirred thoroughly to obtain a mixed solution 3;
   0.75 g of palladium acetylacetonate and 2.4 g of ruthenium acetylacetonate were dissolved in 62.8 g of benzene and stirred thoroughly to obtain a mixed solution 4;
(2) the mixed solution 1 was added to the mixed solution 2 and stirred, the mixture was first dried by evaporation at 99°C for 12 h, and then roasted at 350°C for 4 h, and the obtained composite metal compound was mixed with 30.0 g of an aluminum oxide powder having a particle size of 100 mesh and 4.3 g of sesbania powder to obtain a mixed powder; in this step, an amount of the aluminum oxide powder was 20.8wt% of a total mass of the composite metal compound and the aluminum oxide powder, and an amount of the extrusion promoter was 3.0wt% of a total mass of the composite metal compound and the aluminum oxide powder;
(3) the mixed solution 4 was added dropwise to the mixed solution 3 to obtain a mixed solution 5; and
(4) the mixed powder of step (2) was added to the mixed solution 5 of step (3) and stirred, and the materials required for moulding were mixed and kneaded sufficiently and subjected to extrusion moulding at room temperature by an F-26 twin-screw extruder, wherein the extrusion moulding was performed under an extrusion pressure controlled at 150 N and a screw rotating speed controlled at 30 r/min, and firstly, the extruded materials were dried at 110°C for 8 h, and then roasted at 400°C for 4 h, so as to obtain the dimethylphenylcarbinol hydrogenolysis catalyst denoted as Catalyst B;
in the preparation method, steps (2) and (3) were not required to be performed in a particular order.

According to the X-ray fluorescence spectrometry (XRF) analysis, the composition of Catalyst B is (calculated based on inorganic oxides): 0.15wt% PdO, 0.40wt% RuO₂, 22.0wt% CuO, 2.5wt% NiO, 15.95wt% ZnO, 15.0wt% Al₂O₃, and 44.0wt% SiO₂.

The Catalyst B was subjected to the catalyst reduction test and catalyst performance evaluation. Refer to Example 1 for the specific conditions and operation processes. The result of the hydrogenolysis reaction is shown in Table 1.

### Example 3

This example provides a preparation method for a dimethylphenylcarbinol hydrogenolysis catalyst, and the preparation method comprises the following steps:
(1) Solution preparation:
   To a reaction kettle, 750.3 g of water was first added, and then 113.5 g of copper formate, 39.6 g of nickel formate, and 78.2 g of zinc formate were added, and stirred thoroughly to dissolve to obtain a mixed solution 1;
   189.3 g of urea was dissolved in 441.6 g of water to obtain an aqueous urea solution, and then 70.0 g of alkaline colloidal silica (with a SiO₂ content of 30wt%, a particle size of 30 nm, and a pH of 9.0) was added and stirred thoroughly to obtain a mixed solution 2;
   6.8 g of anilinomethyltrimethoxysilane was added to 248.0g of a propanol nanosilica solution (with a SiO₂ content of 15wt%, and a particle size of 30 nm) and stirred thoroughly to obtain a mixed solution 3;
   0.90 g of palladium acetylacetonate and 1.2 g of ruthenium acetylacetonate were dissolved in 41.9 g of chloroform and stirred thoroughly to obtain a mixed solution 4;
(2) the mixed solution 1 was added to the mixed solution 2 and stirred, the mixture was first dried by evaporation at 95°C for 12 h, and then roasted at 300°C for 8 h, and the obtained composite metal compound was mixed with 50.0 g of an aluminum oxide powder having a particle size of 100 mesh and 4.8 g of sesbania powder to obtain a mixed powder; in this step, an amount of the aluminum oxide powder was 31.2wt% of a total mass of the composite metal compound and the aluminum oxide powder, and an amount of the extrusion promoter was 3.0wt% of a total mass of the composite metal compound and the aluminum oxide powder;
(3) the mixed solution 4 was added dropwise to the mixed solution 3 to obtain a mixed solution 5; and
(4) the mixed powder of step (2) was added to the mixed solution 5 of step (3) and stirred, and the materials required for moulding were mixed and kneaded sufficiently and subjected to extrusion moulding at room temperature by an F-26 twin-screw extruder, wherein the extrusion moulding was performed under an extrusion pressure controlled at 150 N and a screw rotating speed controlled at 30 r/min, and firstly, the extruded materials were dried at 110°C for 12 h, and then roasted at 400°C for 8 h, so as to obtain the dimethylphenylcarbinol hydrogenolysis catalyst denoted as Catalyst C;
in the preparation method, steps (2) and (3) were not required to be performed in a particular order.

According to the X-ray fluorescence spectrometry (XRF) analysis, the composition of Catalyst C is (calculated based on inorganic oxides): 0.18wt% PdO, 0.20wt% RuO₂, 20.0wt% CuO, 8wt% NiO, 16.62wt% ZnO, 25.0wt% Al₂O₃, and 30.0wt% SiO₂.

The Catalyst C was subjected to the catalyst reduction test and catalyst performance evaluation. Refer to Example 1 for the specific conditions and operation processes. The result of the hydrogenolysis reaction is shown in Table 1.

### Example 4

This example provides a preparation method for a dimethylphenylcarbinol hydrogenolysis catalyst, and the preparation method comprises the following steps:
(1) Solution preparation:
   To a reaction kettle, 725.4 g of water was first added, and then 141.8 g of copper formate, 14.8 g of nickel formate, and 72.6 g of zinc formate were added, and stirred thoroughly to dissolve to obtain a mixed solution 1;
   169.9 g of urea was dissolved in 509.7 g of water to obtain an aqueous urea solution, and then 26.3 g of alkaline colloidal silica (with a SiO₂ content of 40wt%, a particle size of 30 nm, and a pH of 9.0) was added and stirred thoroughly to obtain a mixed solution 2;
   9.4 g of anilinomethyltriethoxysilane was added to 196.0 g of an ethanol nanosilica solution (with a SiO₂ content of 15wt%, and a particle size of 30 nm) and stirred thoroughly to obtain a mixed solution 3;
   0.60 g of palladium acetylacetonate and 2.7 g of ruthenium acetylacetonate were dissolved in 65.8 g of benzene and stirred thoroughly to obtain a mixed solution 4;
(2) the mixed solution 1 was added to the mixed solution 2 and stirred, the mixture was first dried by evaporation at 90°C for 12 h, and then roasted at 400°C for 4 h, and the obtained composite metal compound was mixed with 70.0 g of an aluminum oxide powder having a particle size of 100 mesh and 5.0 g of sesbania powder to obtain a mixed powder; in this step, an amount of the aluminum oxide powder was 41.8wt% of a total mass of the composite metal compound and the aluminum oxide powder, and an amount of the extrusion promoter was 3.0wt% of a total mass of the composite metal compound and the aluminum oxide powder;
(3) the mixed solution 4 was added dropwise to the mixed solution 3 to obtain a mixed solution 5; and
(4) the mixed powder of step (2) was added to the mixed solution 5 of step (3) and stirred, and the materials required for moulding were mixed and kneaded sufficiently and subjected to extrusion moulding at room temperature by an F-26 twin-screw extruder, wherein the extrusion moulding was performed under an extrusion pressure controlled at 150 N and a screw rotating speed controlled at 30 r/min, and firstly, the extruded materials were dried at 120°C for 4 h, and then roasted at 450°C for 6 h, so as to obtain the dimethylphenylcarbinol hydrogenolysis catalyst denoted as Catalyst D;
in the preparation method, steps (2) and (3) were not required to be performed in a particular order.

According to the X-ray fluorescence spectrometry (XRF) analysis, the composition of Catalyst D is (calculated based on inorganic oxides): 0.12wt% PdO, 0.45wt% RuO₂, 25.0wt% CuO, 3wt% NiO, 15.43wt% ZnO, 35.0wt% Al₂O₃, and 21.0wt% SiO₂.

The Catalyst D was subjected to the catalyst reduction test and catalyst performance evaluation. Refer to Example 1 for the specific conditions and operation processes. The result of the hydrogenolysis reaction is shown in Table 1.

### Example 5

This example provides a preparation method for a dimethylphenylcarbinol hydrogenolysis catalyst, and the preparation method comprises the following steps:
(1) Solution preparation:
   To a reaction kettle, 774.6 g of water was first added, and then 130.5 g of copper formate, 24.7 g of nickel formate, and 86.1 g of zinc formate were added, and stirred thoroughly to dissolve to obtain a mixed solution 1;
   160.5 g of urea was dissolved in 642.0 g of water to obtain an aqueous urea solution, and then 30.7 g of alkaline colloidal silica (with a SiO₂ content of 30wt%, a particle size of 40 nm, and a pH of 9.0) was added and stirred thoroughly to obtain a mixed solution 2;
   9.6 g of γ-aminopropyltrimethoxysilane was added to 223.9 g of an ethanol nanosilica solution (with a SiO₂ content of 15wt%, and a particle size of 30 nm) and stirred thoroughly to obtain a mixed solution 3;
   1.0 g of palladium acetylacetonate and 3.0 g of ruthenium acetylacetonate were dissolved in 79.8 g of benzene and stirred thoroughly to obtain a mixed solution 4;
(2) the mixed solution 1 was added to the mixed solution 2 and stirred, the mixture was first dried by evaporation at 95°C for 12 h, and then roasted at 300°C for 8 h, and the obtained composite metal compound was mixed with 60.0 g of an aluminum oxide powder having a particle size of 100 mesh and 4.9 g of sesbania powder to obtain a mixed powder; in this step, an amount of the aluminum oxide powder was 37.1wt% of a total mass of the composite metal compound and the aluminum oxide powder, and an amount of the extrusion promoter was 3.0wt% of a total mass of the composite metal compound and the aluminum oxide powder;
(3) the mixed solution 4 was added dropwise to the mixed solution 3 to obtain a mixed solution 5; and
(4) the mixed powder of step (2) was added to the mixed solution 5 of step (3) and stirred, and the materials required for moulding were mixed and kneaded sufficiently and subjected to extrusion moulding at room temperature by an F-26 twin-screw extruder, wherein the extrusion moulding was performed under an extrusion pressure controlled at 150 N and a screw rotating speed controlled at 30 r/min, and firstly, the extruded materials were dried at 120°C for 8 h, and then roasted at 350°C for 8 h, so as to obtain the dimethylphenylcarbinol hydrogenolysis catalyst denoted as Catalyst E;
in the preparation method, steps (2) and (3) were not required to be performed in a particular order.

According to the X-ray fluorescence spectrometry (XRF) analysis, the composition of Catalyst E is (calculated based on inorganic oxides): 0.2wt% PdO, 0.5wt% RuO₂, 23.0wt% CuO, 5.0wt% NiO, 18.3wt% ZnO, 30.0wt% Al₂O₃, and 23.0wt% SiO₂.

The Catalyst E was subjected to the catalyst reduction test and catalyst performance evaluation. Refer to Example 1 for the specific conditions and operation processes. The result of the hydrogenolysis reaction is shown in Table 1.

### Example 6

This example provides a preparation method for a dimethylphenylcarbinol hydrogenolysis catalyst, and the preparation method comprises the following steps:
(1) Solution preparation:
   To a reaction kettle, 644.1 g of water was first added, and then 102.1 g of copper formate, 19.8 g of nickel formate, and 77.8 g of zinc formate were added, and stirred thoroughly to dissolve to obtain a mixed solution 1;
   156.7 g of urea was dissolved in 470.0 g of water to obtain an aqueous urea solution, and then 32.2 g of alkaline colloidal silica (with a SiO₂ content of 40wt%, a particle size of 40 nm, and a pH of 9.0) was added and stirred thoroughly to obtain a mixed solution 2;
   6.8 g of γ-aminopropyltriethoxysilane was added to 208.5 g of a propanol nanosilica solution (with a SiO₂ content of 15wt%, and a particle size of 30 nm) and stirred thoroughly to obtain a mixed solution 3;
   0.8 g of palladium acetylacetonate and 1.8 g of ruthenium acetylacetonate were dissolved in 51.9 g of chloroform and stirred thoroughly to obtain a mixed solution 4;
(2) the mixed solution 1 was added to the mixed solution 2 and stirred, the mixture was first dried by evaporation at 90°C for 12 h, and then roasted at 400°C for 8 h, and the obtained composite metal compound was mixed with 76.0 g of an aluminum oxide powder having a particle size of 100 mesh and 5.0 g of sesbania powder to obtain a mixed powder; in this step, an amount of the aluminum oxide powder was 45.8wt% of a total mass of the composite metal compound and the aluminum oxide powder, and an amount of the extrusion promoter was 3.0wt% of a total mass of the composite metal compound and the aluminum oxide powder;
(3) the mixed solution 4 was added dropwise to the mixed solution 3 to obtain a mixed solution 5; and
(4) the mixed powder of step (2) was added to the mixed solution 5 of step (3) and stirred, and the materials required for moulding were mixed and kneaded sufficiently and subjected to extrusion moulding at room temperature by an F-26 twin-screw extruder, wherein the extrusion moulding was performed under an extrusion pressure controlled at 150 N and a screw rotating speed controlled at 30 r/min, and firstly, the extruded materials were dried at 100°C for 12 h, and then roasted at 400°C for 4 h, so as to obtain the dimethylphenylcarbinol hydrogenolysis catalyst denoted as Catalyst F;
in the preparation method, steps (2) and (3) were not required to be performed in a particular order.

According to the X-ray fluorescence spectrometry (XRF) analysis, the composition of Catalyst F is (calculated based on inorganic oxides): 0.16wt% PdO, 0.3wt% RuO₂, 18.0wt% CuO, 4.0wt% NiO, 16.54wt% ZnO, 38.0wt% Al₂O₃, and 23.0wt% SiO₂.

The Catalyst F was subjected to the catalyst reduction test and catalyst performance evaluation. Refer to Example 1 for the specific conditions and operation processes. The result of the hydrogenolysis reaction is shown in Table 1.

### Comparative Example 1

This comparative example provides a preparation method for a dimethylphenylcarbinol hydrogenolysis catalyst; refer to Example 1. The difference only lies in that: the aqueous urea solution was not used in step (1), i.e., the mixed solution 2 was alkaline colloidal silica (with a SiO₂ content of 30wt%, a particle size of 30 nm, and a pH of 9.0); the obtained dimethylphenylcarbinol hydrogenolysis catalyst was denoted as Catalyst G.

The Catalyst G was subjected to the catalyst reduction test and catalyst performance evaluation. Refer to Example 1 for the specific conditions and operation processes. The result of the hydrogenolysis reaction is shown in Table 1.

### Comparative Example 2

This comparative example provides a preparation method for a dimethylphenylcarbinol hydrogenolysis catalyst; refer to Example 2. The difference only lies in that: the aluminum oxide powder was not used in step (2), i.e., the obtained composite metal compound was directly mixed with 4.3 g of sesbania powder to obtain a mixed powder; the obtained dimethylphenylcarbinol hydrogenolysis catalyst was denoted as Catalyst H.

The Catalyst H was subjected to the catalyst reduction test and catalyst performance evaluation. Refer to Example 1 for the specific conditions and operation processes. The result of the hydrogenolysis reaction is shown in Table 1.

### Comparative Example 3

This comparative example provides a preparation method for a dimethylphenylcarbinol hydrogenolysis catalyst; refer to Example 3. The difference only lies in that: the aminosilane coupling agent (anilinomethyltrimethoxysilane) was not used in step (1), i.e., the mixed solution 3 was a propanol nanosilica solution (with a SiO₂ content of 15wt% and a particle size of 30 nm); the obtained dimethylphenylcarbinol hydrogenolysis catalyst was denoted as Catalyst I.

The Catalyst I was subjected to the catalyst reduction test and catalyst performance evaluation. Refer to Example 1 for the specific conditions and operation processes. The result of the hydrogenolysis reaction is shown in Table 1.

### Comparative Example 4

This comparative example provides a preparation method for a dimethylphenylcarbinol hydrogenolysis catalyst; refer to Example 4. The difference only lies in that: the ethanol nanosilica solution (with a SiO₂ content of 15wt% and a particle size of 30 nm) was not used in step (1), and the alkaline colloidal silica of step (1) (with a SiO₂ content of 40wt%, a particle size of 30 nm, and a pH of 9.0) was increased from 26.3 g to 99.8 g; the obtained dimethylphenylcarbinol hydrogenolysis catalyst was denoted as Catalyst J.

The Catalyst J was subjected to the catalyst reduction test and catalyst performance evaluation. Refer to Example 1 for the specific conditions and operation processes. The result of the hydrogenolysis reaction is shown in Table 1.

### Comparative Example 5

This comparative example provides a preparation method for a dimethylphenylcarbinol hydrogenolysis catalyst; refer to Example 5. The difference only lies in that: palladium acetylacetonate was not used in step (1), and instead, an equal molar amount of ruthenium acetylacetonate was used, i.e., 1.30 g of ruthenium acetylacetonate was added additionally; the obtained dimethylphenylcarbinol hydrogenolysis catalyst was denoted as Catalyst K.

The Catalyst K was subjected to the catalyst reduction test and catalyst performance evaluation. Refer to Example 1 for the specific conditions and operation processes. The result of the hydrogenolysis reaction is shown in Table 1.

### Comparative Example 6

This comparative example provides a preparation method for a dimethylphenylcarbinol hydrogenolysis catalyst; refer to Example 5. The difference only lies in that: ruthenium acetylacetonate was not used in step (1), and instead, an equal molar amount of palladium acetylacetonate was used, i.e., 2.30 g of palladium acetylacetonate was added additionally; the obtained dimethylphenylcarbinol hydrogenolysis catalyst was denoted as Catalyst L.

The Catalyst L was subjected to the catalyst reduction test and catalyst performance evaluation. Refer to Example 1 for the specific conditions and operation processes. The result of the hydrogenolysis reaction is shown in Table 1.

### Comparative Example 7

This comparative example provides a preparation method for a dimethylphenylcarbinol hydrogenolysis catalyst; refer to Example 6. The difference only lies in that: palladium acetylacetonate and ruthenium acetylacetonate were replaced with an equal molar amount of palladium chloride and an equal molar amount of ruthenium chloride, respectively, i.e., 0.46 g of palladium chloride and 0.94 g of ruthenium chloride were dissolved in 28.0 g of water and stirred thoroughly to obtain a mixed solution 4; the obtained dimethylphenylcarbinol hydrogenolysis catalyst was denoted as Catalyst M.

The Catalyst M was subjected to the catalyst reduction test and catalyst performance evaluation. Refer to Example 1 for the specific conditions and operation processes. The result of the hydrogenolysis reaction is shown in Table 1.

The corresponding hydrogenolysis results of the catalysts prepared in the above examples and comparative examples are summarized in Table 1.

**Table 1**

| Item | Dimethylphenylcarbinol Conversion Rate/% | Isopropylbenzene Selectivity/% |
|---|---|---|
| Catalyst A | > 99.9 | 99.3 |
| Catalyst B | >99.9 | 99.5 |
| Catalyst C | >99.9 | 99.4 |
| Catalyst D | >99.9 | 99.5 |
| Catalyst E | >99.9 | 99.2 |
| Catalyst F | >99.9 | 99.5 |
| Catalyst G | 97.2 | 98.6 |
| Catalyst H | 90.7 | 99.0 |
| Catalyst I | 95.3 | 99.6 |
| Catalyst J | 95.1 | 99.2 |
| Catalyst K | 92.5 | 99.4 |
| Catalyst L | 97.9 | 97.1 |
| Catalyst M | 87.3 | 98.2 |

The following conclusions can be drawn from Table 1:
(1) the Catalysts A-F obtained by the preparation method in the present application have good activity and selectivity, indicating that the dimethylphenylcarbinol hydrogenolysis catalyst in the present application has highly dispersed active components and unobstructed pores, and has excellent activity and selectivity when used in isopropylbenzene preparation by the dimethylphenylcarbinol hydrogenolysis;
(2) based on the comparison between Example 1 and Comparative Example 1, it is indicated that the urea added in the preparation process of dimethylphenylcarbinol hydrogenolysis catalyst can improve the mass transfer performance of the catalyst and increase the reaction activity and selectivity;
(3) based on the comparison between Example 2 and Comparative Example 2, it is indicated that the absence of aluminum oxide powder in the preparation process of dimethylphenylcarbinol hydrogenolysis catalyst results in the catalyst having a weak acidity, which is detrimental to the conversion of dimethylphenylcarbinol, and the catalyst has a low activity;
(4) based on the comparison between Example 3 and Comparative Example 3, it is indicated that the aminosilane coupling agent added in the preparation process of dimethylphenylcarbinol hydrogenolysis catalyst improves the dispersion degree of the precious metal, thereby enhances the conversion rate;
(5) based on the comparison between Example 4 and Comparative Example 4, it is indicated that the alcoholic nanosilica solution added in the preparation process of dimethylphenylcarbinol hydrogenolysis catalyst improves the hydrogenolysis reaction activity of the catalyst;
(6) based on the comparison between Example 5 and Comparative Examples 5 and 6, it is indicated that using Ru or Pd singly as the precious metal active component in the dimethylphenylcarbinol hydrogenolysis catalyst results in low hydrogenolysis activity, and moreover, an overly high content of Pd will promote the side reaction of excessive hydrogenation; and
(7) based on the comparison between Example 6 and Comparative Example 6, it is indicated that using palladium acetylacetonate and ruthenium acetylacetonate preferably as the precious metal palladium source and ruthenium source improves the dispersion of Pd and Ru, and thus improves the reaction activity of dimethylphenylcarbinol hydrogenolysis catalyst.

Although specific process equipment and process flow of the present application are illustrated in terms of the examples in the present application, the present application is not limited to the specific process equipment and process flow, i.e., the present application does not necessarily rely on the specific process equipment and process flow to be implemented. It should be clear to those skilled in the art that improvement of the present application, equivalent substitution of raw materials of the product of the present application, addition of auxiliary ingredients, selection of specific methods, etc., shall all fall within the protection scope and disclosure scope of the present application.

## Claims

1. A dimethylphenylcarbinol hydrogenolysis catalyst, wherein provided that the total weight of the dimethylphenylcarbinol hydrogenolysis catalyst is 100wt% and calculated based on inorganic oxides, the dimethylphenylcarbinol hydrogenolysis catalyst comprises the following components:
| | |
|---|---|
| PdO | 0.01-0.3wt%; |
| RuO₂ | 0.05-0.8wt%; |
| CuO | 15.0-25.0wt%; |
| ZnO | 15.0-25.0wt%; |
| NiO | 2.0-10.0wt%; |
| Al₂O₃ | 15.0-40.0wt%; and |
| SiO₂ | 20.0-45.0wt%. |

2. The dimethylphenylcarbinol hydrogenolysis catalyst according to claim 1, wherein a molar ratio of Pd to Ru is 1: (1-10), a molar ratio of Cu to Zn is 1: (0.5-1.0), and a molar ratio of Cu to Ni is 1: (0.1-0.5).

3. The dimethylphenylcarbinol hydrogenolysis catalyst according to claim 1 or 2, wherein the dimethylphenylcarbinol hydrogenolysis catalyst has a hollow ring shape with an outer diameter of 3-5 mm, an inner diameter of 1-3 mm, and a length of 3-8 mm.

4. A preparation method for the dimethylphenylcarbinol hydrogenolysis catalyst according to any one of claims 1-3, comprising the following steps:
(1) preparing a mixed solution 1 of a copper-containing compound, a zinc-containing compound, and a nickel-containing compound; adding colloidal silica to an aqueous urea solution to obtain a mixed solution 2; adding an aminosilane coupling agent to an alcoholic nanosilica solution to obtain a mixed solution 3; and preparing a mixed solution 4 of a palladium-containing compound and a ruthenium-containing compound;
(2) adding the mixed solution 1 to the mixed solution 2, sequentially performing stirring, evaporating to dry, and roasting, and mixing a composite metal compound obtained with an aluminum oxide powder and an extrusion promoter to obtain a mixed powder;
(3) adding the mixed solution 4 dropwise to the mixed solution 3 to obtain a mixed solution 5; and
(4) adding the mixed powder of step (2) to the mixed solution 5 of step (3), and sequentially performin mixing, extrusion moulding, drying, and roasting to obtain the dimethylphenylcarbinol hydrogenolysis catalyst;
wherein steps (2) and (3) are not required to be performed in a particular order.

5. The preparation method according to claim 4, wherein, in the mixed solution 1 of step (1), the copper-containing compound comprises copper formate and/or copper acetate;
optionally, in the mixed solution 1 of step (1), the zinc-containing compound comprises zinc formate and/or zinc acetate;
optionally, in the mixed solution 1 of step (1), the nickel-containing compound comprises nickel formate and/or nickel acetate;
optionally, in the mixed solution 1 of step (1), metal ions have a molar concentration of 1.0-2.0 mol/L;
optionally, the aqueous urea solution of step (1) has a mass concentration of 15-30wt%; optionally, the colloidal silica of step (1) is an alkaline silica sol;
optionally, for the alkaline silica sol, a content of SiO₂ is 30-40wt%, a particle size is 20-40 nm, and a pH is 8.0-10.0;
optionally, for the alcoholic nanosilica solution of step (1), a content of SiO₂ is 15-20wt%, and a particle size is 15-30 nm;
optionally, an alcoholic solvent in the alcoholic nanosilica solution of step (1) comprises any one or a combination of at least two of methanol, ethanol or propanol.

6. The preparation method according to claim 4 or 5, wherein the aminosilane coupling agent of step (1) comprises any one or a combination of at least two of γ-aminopropyltrimethoxysilane, γ-aminopropyltriethoxysilane, anilinomethyltrimethoxysilane, or anilinomethyltriethoxysilane;
optionally, in the preparation for the mixed solution 3 of step (1), a mass ratio of the aminosilane coupling agent to the alcoholic nanosilica solution is controlled to be 1: (20-50);
optionally, in the mixed solution 4 of step (1), the palladium-containing compound comprises palladium acetylacetonate;
optionally, in the mixed solution 4 of step (1), the ruthenium-containing compound comprises ruthenium acetylacetonate;
optionally, a solvent in the mixed solution 4 of step (1) comprises any one or a combination of at least two of benzene, toluene, or chloroform.

7. The preparation method according to any one of claims 4-6, wherein, in step (2), a molar ratio of the metal ions in the mixed solution 1 to urea in the mixed solution 2 is controlled to be 1: (2.0-3.0);
optionally, the evaporating to dry of step (2) is performed at a temperature of 80-100°C;
optionally, the evaporating to dry of step (2) is performed for a period of 4-24 h;
optionally, the roasting of step (2) is performed at a temperature of 250-400°C;
optionally, the roasting of step (2) is performed for a period of 2-8 h.

8. The preparation method according to any one of claims 4-7, wherein the aluminum oxide powder of step (2) has a particle size of 80-150 mesh;
optionally, an amount of the aluminum oxide powder of step (2) is 15-40wt% of a total mass of the composite metal compound and the aluminum oxide powder;
optionally, the extrusion promoter of step (2) comprises sesbania powder;
optionally, an amount of the extrusion promoter of step (2) is 2-5wt% of a total mass of the composite metal compound and the aluminum oxide powder.

9. The preparation method according to any one of claims 4-8, wherein process conditions for the extrusion moulding of step (4) comprise: mixing and kneading materials used for moulding thoroughly, and performing extrusion moulding at room temperature using an F-26 twin-screw extruder;
optionally, the extrusion moulding has an extrusion pressure of 100-200 N and a screw rotating speed of 10-50 r/min;
optionally, the drying of step (4) is performed at a temperature of 100-120°C;
optionally, the drying of step (4) is performed for a period of 4-12 h;
optionally, the roasting of step (4) is performed at a temperature of 300-450°C;
optionally, the roasting of step (4) is performed for a period of 2-8 h.

10. The preparation method according to any one of claims 4-9, wherein the preparation method comprises the following steps:
(1) preparing solutions:
preparing a mixed solution 1 of a copper-containing compound, a zinc-containing compound, and a nickel-containing compound; wherein the copper-containing compound comprises copper formate and/or copper acetate, the zinc-containing compound comprises zinc formate and/or zinc acetate, and the nickel-containing compound comprises nickel formate and/or nickel acetate; and metal ions in the mixed solution 1 have a molar concentration of 1.0-2.0 mol/L;
adding alkaline colloidal silica to an aqueous urea solution having a mass concentration of 15-30wt% to obtain a mixed solution 2; wherein for the alkaline silica sol, a content of SiO₂ is 30-40wt%, a particle size is 20-40 nm, and a pH is 8.0-10.0;
adding an aminosilane coupling agent to an alcoholic nanosilica solution, wherein a mass ratio of the aminosilane coupling agent to the alcoholic nanosilica solution is controlled to be 1: (20-50), to obtain a mixed solution 3; wherein for the alcoholic nanosilica solution, a content of SiO₂ is 15-20wt% and a particle size is 15-30 nm; an alcoholic solvent in the alcoholic nanosilica solution comprises any one or a combination of at least two of methanol, ethanol, or propanol; the aminosilane coupling agent comprises any one or a combination of at least two of γ-aminopropyltrimethoxysilane, γ-aminopropyltriethoxysilane, anilinomethyltrimethoxysilane, or anilinomethyltriethoxysilane;
preparing a mixed solution 4 of a palladium-containing compound and a ruthenium-containing compound; wherein the palladium-containing compound comprises palladium acetylacetonate, the ruthenium-containing compound comprises ruthenium acetylacetonate, and a solvent in the mixed solution 4 comprises any one or a combination of at least two of benzene, toluene, or chloroform;
(2) adding the mixed solution 1 to the mixed solution 2, and stirring the mixture, wherein a molar ratio of the metal ions in the mixed solution 1 to urea in the mixed solution 2 is controlled to be 1:(2.0-3.0), firstly evaporating the mixture at 80-100°C for 4-24 h and then roasting at 250-400°C for 2-8 h, and mixing a composite metal compound obtained with an aluminum oxide powder having a particle size of 80-150 mesh and an extrusion promoter to obtain a mixed powder; wherein an amount of the aluminum oxide powder is 15-40wt% of a total mass of the composite metal compound and the aluminum oxide powder, and an amount of the extrusion promoter is 2-5wt% of a total mass of the composite metal compound and the aluminum oxide powder;
(3) adding the mixed solution 4 dropwise to the mixed solution 3 to obtain a mixed solution 5; and
(4) adding the mixed powder of step (2) to the mixed solution 5 of step (3) and stirring them, mixing and kneading materials used for moulding thoroughly, and performing extrusion moulding at room temperature using an F-26 twin-screw extruder, wherein the extrusion moulding is performed under an extrusion pressure controlled at 100-200 N and a screw rotating speed controlled at 10-50 r/min, and firstly drying at 100-120°C for 4-12 h and then roasting at 300-450°C for 2-8 h to obtain the dimethylphenylcarbinol hydrogenolysis catalyst;
wherein steps (2) and (3) are not required to be performed in a particular order.

11. Use of a dimethylphenylcarbinol hydrogenolysis catalyst, wherein the dimethylphenylcarbinol hydrogenolysis catalyst according to any one of claims 1-3 or the dimethylphenylcarbinol hydrogenolysis catalyst obtained by the preparation method according to any one of claims 4-10 is applied to a reaction of preparing isopropylbenzene by dimethylphenylcarbinol hydrogenolysis.
